# EUROPÄISCHE PATENTSCHRIFT

(11) **EP 1 133 697 B1**
(45) Veröffentlichungstag und Bekanntmachung des Hinweises auf die Patenterteilung: **07.08.2002**
(21) Anmeldenummer: 99958116.8
(22) Anmeldetag: 23.11.1999
(51) Int. Cl.: G01N 33/68, G01N 33/533

(54) **VERFAHREN ZUR MODIFIKATION UND IDENTIFIKATION FUNKTIONELLER STELLEN IN PROTEINEN**
METHOD FOR MODIFYING AND IDENTIFYING FUNCTIONAL SITES IN PROTEINS
PROCEDE DE MODIFICATION ET D'IDENTIFICATION DE SITES FONCTIONNELS DANS DES PROTEINES

(30) Priorität: 24.11.1998 DE 19854196
(43) Veröffentlichungstag der Anmeldung: 19.09.2001
(73) Patentinhaber: Xerion Pharmaceuticals GmbH, 82152 Martinsried (DE)
(72) Erfinder: ILAG, L., Leodevico, D-81369 München (DE); NG, Jocelyn, H., D-81369 München (DE)
(74) Vertreter: Bösl, Raphael, Dr. rer. nat., Dipl.-Chem.
(86) Internationale Anmeldenummer: EP9909052
(87) Internationale Veröffentlichungsnummer: WO0031544

(56) Entgegenhaltungen:
- WO-A-99/20190
- WO-A-99/41607
- HEGYI, GYOERGY ET AL: "Intrastrand Cross-Linked Actin between Gln-41 and Cys-374. I. Mapping of Sites Cross-Linked in F-actin by N-(4-azido-2- nitrophenyl) Putrescine" BIOCHEMISTRY (22-12-1998), 37(51), 17784-17792 , XP002132757
- JAY D G: "SELECTIVE DESTRUCTION OF PROTEIN FUNCTION BY CHROMOPHORE-ASSISTED LASER INACTIVATION" PROCEEDINGS OF THE NATIONAL ACADEMY OF SCIENCES OF USA,US,NATIONAL ACADEMY OF SCIENCE. WASHINGTON, Bd. 85, Nr. 15, August 1988 (1988-08), Seiten 5454-5458, XP000857275 ISSN: 0027-8424 in der Anmeldung erwähnt
- WANG FENG-SONG; JAY DANIEL G: "Chromophore-assisted laser inactivation (CALI): Probing protein function in situ with a high degree of spatial and temporal resolution" TRENDS IN CELL BIOLOGY, Bd. 6, 1996, Seiten 442-445, XP000890066 in der Anmeldung erwähnt
- BEERMANN ANKE E L; JAY DANIEL G: "Chromophore-assisted laser inactivation of cellular proteins" METHODS IN CELL BIOLOGY, Bd. 44, 1994, Seiten 715-732, XP000889671 in der Anmeldung erwähnt
- R F STEINER, S ALBAUGH, C FENSELAU, C MURPHY, M VESTLING: "A Mass Spectrometry Method for Mapping the Interface Topography of Interacting Proteins, Illustrated by the Melittin-Calmodulin System" ANALYTICAL BIOCHEMISTRY, Bd. 196, 1991, Seiten 120-125, XP002132758 in der Anmeldung erwähnt

## Beschreibung

Die Erfindung betrifft ein Verfahren zur gezielten Modifikation und Identifikation funktioneller Stellen in einem Zielprotein. Durch Funktionsverlust/Funktionsgewinn des Proteins und die anschließende Bestimmung der modifizierten Aminosäuren kann eine Korrelation zwischen der (den) spezifischen Aminosäure(n) und ihrer Funktion im Protein bzw. ihrer Funktion als Ligandenbindungsstelle erstellt werden (Epitop-Kartierung). Das Verfahren beruht auf der kombinierten Verwendung von chromophor-unterstützter Laserinaktivierung (chromophore-assisted laser inactivation; CALI) und Massenspektrometrie bzw. Proteinsequenzierung. Die Erfindung betrifft auch eine Vorrichtung zur Durchführung dieses Verfahrens.

Die Techniken der rekombinanten DNA und der Proteinherstellung erleichtern das tiefere Verständnis der Beziehungen zwischen Proteinstruktur und Proteinfunktion. Methoden der Zufallsmutagenese, gezielte genetische Selektion und Screening-Verfahren ermöglichen einen raschen Informationsgewinn auf Proteinebene, besitzen aber den Nachteil, daß bestimmte funktionelle Domänen des Proteins sehr tolerant gegenüber Aminosäuresubstitutionen sind, daß manche Mutagenesereagentien bestimmte DNA-Sequenzen bevorzugt angreifen und mögliche Mutationen begrenzt sind. Somit ist die Aussagekraft dieser Methoden begrenzt. Als Alternative bieten ortsspezifische Mutagenesemethoden einen direkteren und gezielteren Weg zur Identifikation von Aminosäuren, die für die biologische Funktion wichtig sind, wie z.B. das Alanin-Scanning-Mutagenese-Verfahren. Bei diesem Verfahren erfolgt eine systematische Mutation jeder Aminosäure zu Alanin und die Funktion wird bestimmt. Jeder Funktionverlust wird in Beziehung zu der speziellen Aminosäure gesetzt. Diese Technik kann auf Proteine ohne dazugehörige dreidimensionale Struktur und ohne unbekannte funktionelle Domänen angewendet werden. Außerdem wird dabei jeweils nur eine Aminosäure modifiziert, weshalb spezielle Funktionen, an denen mehrere Aminosäuren beteiligt sind, nicht ermittelt werden. Ferner wird das Verfahren mit zunehmender Molekülgröße aufwendiger. Dies ist bei der Analyse der Funktion von Genprodukten im Zusammenhang mit Krankheiten nachteilig, da diese oft große Proteine sind, die multiple strukturelle Domänen enthalten.

Des weiteren wurden andere rekombinante DNA-Techniken zur Epitopkartierung und zur Kartierung von Ligandenbindungsstellen verwendet, bei denen Random-Antigenfragmente mit auf der DNA-Ebene eingeführten Mutationen exprimiert werden (vgl. J. Immunol. Meth., 141, 245-252, 1991, J. Immunol. Meth., 180, 53-61, 1995, Virology, 198, 346-349, 1994). Jüngst wurde das Phagen-Display von Random-Peptidbanken verwendet, um Epitope zu kartieren (J. Immunol., 153, 724-729, 1994, PNAS, 93, 1997-2001, 1996).

Eine weitere Variante der Epitopkartierung von Proteinen ist die rasche automatische Synthese von ausgewählten Peptiden (J. Endocrinol., 145, 169-174, 1995) und die Verwendung von kombinatorischen Peptidbanken (FEBS Letters, 352, 167-170, 1994). Obwohl diese Verfahren bei linearen Epitopen zuverlässig sind, waren sie mit nicht-linearen oder diskontinuierlichen Epitopen nicht erfolgreich. Auch die Variante der Verwendung überlappender Peptide war bei diskontinuierlichen Epitopen nicht zufriedenstellend. Dabei werden einzelne Peptide oder Peptidgemische auf die Bindung an den Liganden mittels ELISA abgesucht, wobei freie und gebundene Peptide um den Liganden (z.B. Antikörper) konkurrieren. Dieses Verfahren ist jedoch aufwendig und zeitraubend.

Gemäß einem weiteren Ansatz wurde die Kombination aus Proteinmodifikation und Massenspektrometrie verwendet (Anal. Biochem., 196, 120-125, 1991). Hierbei wird der Ligand an den Rezeptor gebunden und der Komplex mit Essigsäureanhydrid modifiziert, wodurch die Lysinreste acetyliert werden. Die proteolytischen Spaltansätze beider Proteine werden massenspektrometrisch analysiert und mit den entsprechenden Fragmenten des unbehandelten Komplexes verglichen. Die modifizierten Lysine können leicht nachgewiesen werden, wobei diese Lysine nicht im Komplex geschützt sind und die nicht-modifizierten Lysine ein Teil der Wechselwirkung zwischen Ligand und Rezeptor sind. Diese Technik ist somit auf Wechselwirkungen unter Beteiligung von Lysinresten beschränkt. Eine weitere Variante beruht auf der differentiellen Proteolyse (Protein Science, 4, 1088-1099, 1995), wobei nach Komplexbildung proteolytisch empfindliche Stellen proteaseresistent werden.

Beschrieben wurde weiterhin die massenspektrometrische Identifikation einer proteolytischen Spaltung von freiem Peptidantigen im Vergleich zu dem Muster aus dem an einen Antikörper gebundenen Peptidantigen. Die Identifikation erfolgte mit ²⁵²Cf-Plasmadesorptionsmassenspektrometrie (PNAS 87, 9848-9852, 1990). Beschrieben ist ferner eine Kombination aus Immunpräzipitation und matrixgestützter Laserdesorptionsmassenspektrometrie (MALDI-MS) (PNAS, 93, 4020-4024, 1996). Dabei wird ein Antigenprotein in kleinere Fragmente gespalten und mit einem interessierenden Antikörper gefällt. Die immunpräzipitierten Peptide werden mit MALDI-MS identifiziert und die Antikörper-bindende Region wird bestimmt. Ferner wurden bei diesem Verfahren proteolytisch gespaltene Peptide mittels Affinitäts-Kapillarelektrophorese getrennt und mit Elektrospray-Massenspektrometrie (ACE-MS) identifiziert (Anal. Chem., 69, 3008-3014, 1997). Nach der Injektion des Peptidgemisches wird der Antikörper injiziert. Peptide, die an den Antikörper binden, werden eingefangen und wandern daher nicht. Nach dem Subtraktions-Screening-Verfahren wird das gebundene Peptid untersucht, um den Epitoprest auf dem Peptid zu bestimmen. Diese Technik ist jedoch auf lineare Epitope beschränkt und kann nicht auf diskontinuierliche Epitope angewendet werden.

Wang, F.-S. und Jay, D.G. (1996) Trends in Cell Biology 6, 442-445, ist ein Übersichtsartikel, in dem die vorbekannte sog. CALI (Chromophore-assisted laser inactivation)-Technologie ganz allgemein für den Zweck der Identifizierung der molekularen oder zellulären Funktion von Proteinen beschrieben wird. Es wird jedoch weder beschrieben noch vorgeschlagen, CALI für die Identifizierung von funktionellen Stellen in Proteinen oder sogar spezifischen Aminosäuren, die für eine bestimmte Funktion der Proteine wichtig ist, einzusetzen.

Der vorliegenden Erfindung liegt daher die Aufgabe zugrunde, die Probleme des genannten Standes der Technik zu überwinden. Es soll ein Verfahren bereitgestellt werden, mit dem beliebige funktionelle Stellen in beliebigen Proteinen identifiziert werden können. Bevorzugt sollen Stellen, die an einer Ligandenwechselwirkung beteiligt sind, und Epitope identifiziert werden können. Insbesondere soll das erfindungsgemäße Verfahren auf nicht-lineare und diskontinuierliche Epitope und ohne Kenntnis der Raumstruktur eines Proteins angewendet werden können. Erfindungsgemäß soll auch eine Bestimmung der Proteinfunktion ohne Inaktivierung des Moleküls möglich sein. Das Verfahren soll außerdem einfach in der Anwendung, rasch durchführbar und automatisierbar sein. Ferner soll auch eine Vorrichtung zur einfachen Durchführung des erfindungsgemäßen Verfahrens bereitgestellt werden.

Gelöst wird diese Aufgabe durch ein Verfahren zur Identifikation einer oder mehrerer funktioneller Stellen in einem Protein, das dadurch gekennzeichnet ist, daß man
a) das Zielprotein mit einem mit einem laseraktivierbaren Marker (Tag) verknüpften Bindungspartner (BP-Tag) in Kontakt bringt, wobei sich ein Komplex aus Zielprotein und BP-Tag bildet,
b) den Komplex aus Zielprotein und BP-Tag mit Laserlicht bestrahlt, wodurch freie Radikale erzeugt werden, die das gebundene Zielprotein an der Bindungsstelle selektiv verändern, und
c) die selektiv veränderte Region des Proteins durch eine Kombination aus Proteinspaltung und Massenspektrometrie identifiziert,
sowie durch eine Vorrichtung zur Durchführung des Verfahrens.

Es wurde überraschenderweise gefunden, daß die Kombination der CALI-Technik mit der Massenspektrometrie eine zuverlässige und rasche Identifizierung von funktionellen Stellen auf Proteinen erlaubt. Erfindungsgemäß wird ein Zielprotein mittels CALI modifiziert, wodurch es inaktiviert wird. Anschließend wird die modifizierte Region des Proteins mittels Massenspektrometrie bestimmt. Bevorzugt werden Tandem-MS und/oder de novo-Sequenzierung verwendet. Dadurch kann auf einfache Weise eine Korrelation zwischen der Aminosäure und ihrer biologischen Funktion erstellt werden. Durch diese Korrelation zwischen Struktur und Funktion lassen sich Aussagen z.B. über die Korrelation zwischen einer bestimmten Stoffwechselaktivität und der entsprechenden Stelle im Molekül, über krankhaft veränderte Proteine, krebsfördernde Proteine, etc. treffen. So können auch gezielt unerwünschte (z.B. pathologische) Proteine inaktiviert werden.

Das Zielprotein wird zunächst mit einem Bindungspartner unter den Bedingungen in Kontakt gebracht, daß eine Komplexbildung stattfindet, die Proteine aber nicht denaturiert werden. Idealerweise entsprechen die Bedingungen den physiologischen Bedingungen der zellulären Umgebung der Proteine. Der Bindungspartner ist mit einem laseraktivierbaren Marker verknüpft. Der laseraktivierbare Marker kann jeder beliebige Marker sein, der sich zur kovalenten oder nicht-kovalenten Anknüpfung an einen Bindungspartner, i.e. eine Aminosäuresequenz, eignet und so aktivierbar ist, daß er freie Radikale erzeugen kann. Die Aktivierung des Makers erfolgt bevorzugt mit Laserlicht, kann aber auch durch Peroxidasen (System Wasserstoffperoxid/Meerrettichperoxidase) erfolgen. Beispiele für derartige Marker sind AMCA-S, AMCA, BODIPY und Varianten davon, Cascade Blue, Cl-NERF, Dansyl, Dialkylaminocumarin, 4',5'Dichlor-2',7'-dimethoxyfluorescein, DM-NERF, Eosin, Eosin F3S, Erythrosin, Hydroxycumarin, Isosulfan blue, Lissamine Rhodamin B, Malachitgrün, Methoxycumarin, Naphthofluorescein, NBD, Oregon Green 488, 500, 514, PyM-PO, Pyrol, Rhodamin 6G, Rhodamin Green, Rhodamin Red, Rhodol green, 2',4',5',7'-Tetrabromsulfonfluorescein, Tetramethylrhodamin, Texas Red oder X-Rhodamin. Bevorzugt ist der Marker Malachitgrünisothiocyanat, Fluoresceinisothiocyanat, oder 4',5'-bis-(1,3,2-dithioarsolan-2-yl)fluorescein. Die Bestrahlung erfolgt mit Laserlicht einer Wellenlänge, die von dem jeweiligen Chromophor absorbiert wird.

Der Bindungspartner kann jeder beliebige Bindungspartner für das entsprechende Protein sein. Er ist bevorzugt scFv, Fab, ein Diabody, ein immunglobulinartiges Molekül, ein Peptid, RNA, DNA, PNA oder ein kleines organisches Molekül.

Bevorzugt stammt der Bindungspartner aus einer kombinatorischen Bank. Dies kann jede beliebige kombinatorische Bank sein, z.B. Proteinbank, Peptidbank, cDNA-Bank, mRNA-Bank, Bank mit organischen Molekülen, scFv-Bank mit Immunglobulinsuperfamilie, Proteindisplay-Bank etc.. In den Banken können präsentiert sein: alle Arten von Proteinen, z.B. Strukturproteine, Enzyme, Rezeptoren, Liganden, alle Arten von Peptiden einschließlich Modifikationen, DNAs, RNAs, Kombinationen von DNAs und RNAs, Hybride von Peptiden und RNA oder DNA, alle Arten von organischen Molekülen, z.B. Steroide, Alkaloide, Naturstoffe, synthetische Stoffe etc. Die Präsentation kann auf verschiedene Arten erfolgen, z.B. als Phagen-Display-System (z.B. filamentöse Phagen wie M13, fl, fd etc., lambda-Phagen-Display, virales Display etc.), Präsentation auf Bakterienoberflächen, Ribosomen etc.

Mit der CALI-Technik werden Zielproteine direkt und gezielt inaktiviert (vgl. PNAS, 85, 5454-5458, 1988; Trends in Cell Biology, 6, 442-445, 1996). Mittels CALI können Bindungsreagentien wie Antikörper oder andere Liganden in funktionsblockierende Moleküle umgewandelt werden. Dabei wird ein Bindungspartner (BP) z.B. mit dem Farbstoff Malachitgrün (MG) verknüpft. Bei Bestrahlung mit Laserlicht mit einer Wellenlänge, die nicht signifikant von den zellulären Komponenten absorbiert wird, erzeugt dieser Farbstoff freie Radikale. Zur Bestimmung wird der mit MG verknüpfte BP (=BP-MG) mit der interessierenden Proteinprobe inkubiert. Der zu inaktivierende Bereich wird ausgewählt und mit einem Laserstrahl mit 620 nm bestrahlt. Dieses Licht wird von MG absorbiert, es entstehen kurzlebige freie Radikale, die selektiv die an den BP-MG gebundenen Proteine in einem Radius von 15 Å durch eine irreversible chemische Modifikation inaktivieren. Dieses System kann für in vitro- und in vivo-Assays sowie für intra- und extrazelluläre Zielmoleküle verwendet werden.

Das so inaktivierte Protein wird nun gespalten. Hierzu kann eine Protease verwendet werden, die spezifisch nach einem Rest spaltet, z.B. Lys-C, Glu-C, Asp-N. Beispiele hierfür sind Trypsin, Chymotrypsin, Papain, etc. Auch eine chemische Spaltung ist möglich, z.B. mit Bromcyan (spezifisch für Met), 3-Brom-3-methyl-2-(2-nitrophenylmercapto)-3H-indol (BNPS-Skatol; spezifisch für Trp), 2-Nitro-5-thiocyanobenzoesäure (spezifisch für Cys) und Fe-EDTA.

Dann kann mittels Massenspektrometrie und Vergleich mit dem unbehandelten Zielprotein die Auswertung der modifizierten Aminosäuren vorgenommen werden.

Die jüngsten Entwicklungen in der Massenspektrometrie führten zu einer raschen Identifikation von Proteinen (PNAS USA, 93, 14440-14445, 1996). Sobald das Zielprotein mittels CALI inaktiviert wurde, können die modifizierten Aminosäuren des inaktivierten Proteins, die vermutlich für die Inaktivierung verantwortlich sind, mittels Tandem-Massen-spektrometrie und ggf. de novo-Sequenzierung identifiziert werden (Rapid Commun. Mass Spectrom., 11, 1015-1024, 1997; Rapid Commun. Mass Spectrom., 11, 1067-1075, 1997)

Die Identität der erfindungsgemäß mit einem Bindungspartner komplexierten Proteine wird durch eine Kombination aus Proteinspaltung und Massenspektrometrie, ggf. de-novo-Sequenzierung identifiziert.

Dabei wird das so behandelte, mit einem Marker versehene Protein zunächst von dem Zielprotein durch eine Elektrophorese oder durch eine Chromatographie getrennt. Das isolierte und modifizierte Protein wird dann entweder chemisch oder proteolytisch nach den vorstehend beschriebenen Verfahren gespalten. Dies kann entweder im Gel (d.h. durch direkte Elution des Zielproteins vom Gel nach der Trennung, gefolgt von einer anschließenden Proteintrennung) oder in Lösung erfolgen. Verfahren zur Spaltung im Gel sind bekannt und z.B. in Advanced Methods in Biological Mass Spectrometry, EMBL-Laboratory, Heidelberg, 1997 oder in Shevchenko, A., et al., Anal. Chem. 68:850-858, 1996, beschrieben.

Die MALDI-Analyse wird in an sich bekannter Weise durchgeführt.

Für die Nanoelektrospray-Analyse (nanoES) müssen die tryptischen Peptide aus den Gelstücken extrahiert werden. Dazu werden die Gelstücke nacheinander mit Ammoniumbicarbonat, Acetonitril, verdünnter Ameisensäure und wieder mit Acetonitril gewaschen. Die Überstände werden vereinigt und in einer Vakuumzentrifuge getrocknet. Die Probe wird in 80%iger Ameisensäure aufgelöst, rasch mit Wasser verdünnt und dann entsalzt.

Die massenspektrometrische Analyse kann auf verschiedene, an sich bekannte Arten durchgeführt werden, z.B. mit einer Ionisierungsquelle wie einem Elektrospray (Chapman, J.R., et al., Methods in Molecular Biology, 61, JR Chapman Hrsg., Humana Press Inv. Totowa NJ, USA, 1996) einschließlich Nanoelektrospray (Wilm. M. und Mann, M., Anal. Chem. 68, 1-8, 1996) und matrixunterstützter Laserdesorption und Ionisierung (MALDI) (Siuzdak, G. Mass Spectrometry for Biotechnology, Academic Press Inc. 1996) oder mit einer Kombination aus Massenanalysatoren wie Quadrupol, Flugzeit, Magnetsektor, Fourier-Transformations-Ionenzyklotron-Resonanz und Quadrupol-Ioneneinfang.

Wenn die Peptide aus dem Spaltansatz nicht ausreichen, die Identität der untersuchten Stelle im Protein eindeutig zu identifizieren, kann durch eine weitere Fragmentierung im Massenspektrometer wie z.B. durch Zerfall nach der Quelle in MALDI-TOF, MS/MS (Tandem-Massenspektrometrie), MSⁿ eine weitere Sequenzinformation erhalten werden. Zusätzlich können die Proteine durch de novo-Sequenzierung identifiziert werden.

Da bei CALI bestimmte Aminosäuren des Zielproteins photochemisch modifiziert werden, kann die MS in zwei Stufen angewendet werden, um zu identifizieren, welche Aminosäuren auf welche Weise modifiziert wurden. Das Verfahren kann mit niedriger und hoher Auflösung durchgeführt werden.

Bei niedriger Auflösung kann zuerst durch Peptidmassenkartierung (Anal. Chem., 69, 4741-4750, 1997; Biochem. Soc. Transactions, 24, 893-896, 1996; Anal. Chem., 69, 1706-1714, 1997) bestimmt werden, welche Segmente des Proteins modifiziert wurden. In der zweiten Stufe kann bei höherer Auflösung mittels Tandem-Massenspektrometrie und/oder de novo-Sequenzieren an ausgewählten Peptiden der Ort der Modifikationen bestimmt werden. Mit einer Massenspektrometer-Konfiguration, die Massen von 0 bis zu 0,03 Dalton auflösen kann (Rapid Commun. Mass Spectrom., 11, 1015-1024) (qQTOF und andere Massenanalysatoren) ist es möglich, zu bestimmen, welche speziellen Aminosäuren mit CALI modifiziert wurden; es kann sogar der Typ der Modifikation aus dem inkrementellen Massenzuwachs- bzw. - verlust bestimmt werden.

Die durch CALI erzeugten freien Radikale führen zu einer Modifikation der oxidationsempfindlichen Aminosäureseitenketten wie His, Met, Cys und Trp. Möglicherweise werden auch andere Aminosäureseitenketten modifiziert. Dadurch ergeben sich signifikante Veränderungen der Masse, da Sauerstoff addiert wurde. Der Vergleich der Peptidfragmente nach der Spaltung zwischen CALI-behandelten Fragmenten und unbehandelter Probe führt zu einer ungefähren Lokalisierung der modifizierten Aminosäuren. Genauer werden diese Aminosäuren durch de-novo-Sequenzierung identifiziert.

Es ist nicht notwendig, daß die genaue Art der Modifikation bekannt ist. Das erfindungsgemäße Verfahren erlaubt die Erkennung der Modifikation aus dem Unterschied der behandelten und der unbehandelten Probe. Der Vorteil dieses Verfahrens liegt darin, daß damit diskontinuierliche Epitope aufgeklärt werden können, selbst wenn die dreidimensionale Struktur des Proteins nicht bekannt ist.

Zur Aufklärung der durch CALI induzierten Modifikationen können auch andere Verfahren wie Parent-Ion-Scans (J. Mass Spectrom, 32, 94-98, 1997) verwendet werden, wodurch die Geschwindigkeit der Analyse erhöht wird. Hiermit werden nur die durch CALI veränderten Peptide erfaßt.

Sollte wegen limitierter Peptidfragmentierung oder zweifelhafter Massenzuordnung nicht genügend Information erhalten werden können, so kann eine de-novo-Massensequenzierung (Rapid Commun. Mass Spectreom., 11, 1015-1024, 1997) durchgeführt werden. Bei diesem Verfahren werden die Peptide mit ¹⁸O markiert. Dazu wird die tryptische Spaltung im Gel mit einem Spaltungspuffer, der 50% (Vol./Vol.) durch Mikrodestillation gereinigtes H₂¹⁸O enthält, durchgeführt. Bevorzugt wird ein QqTOF-Massenspektrometer verwendet. Die Ablesung der Dubletts aufgrund des 1:1-Verhältnisses von ¹⁶O/¹⁸O ermöglicht klare Ergebnisse. Die Massendifferenz des Dubletts ist ein Anzeichen für den Ladungszustand des speziellen Peptids. Unter Verwendung verschiedener Ladungszustände können bis zu 15 Aminosäuren in einer Sequenz für ein gegebenes Peptid abgelesen werden. Der Vergleich der ¹⁸O-markierten Spektren der unbehandelten und mit CALI modifizierten Proteine erlaubt eine eindeutige Aussage über die modifizierten Aminosäuren.

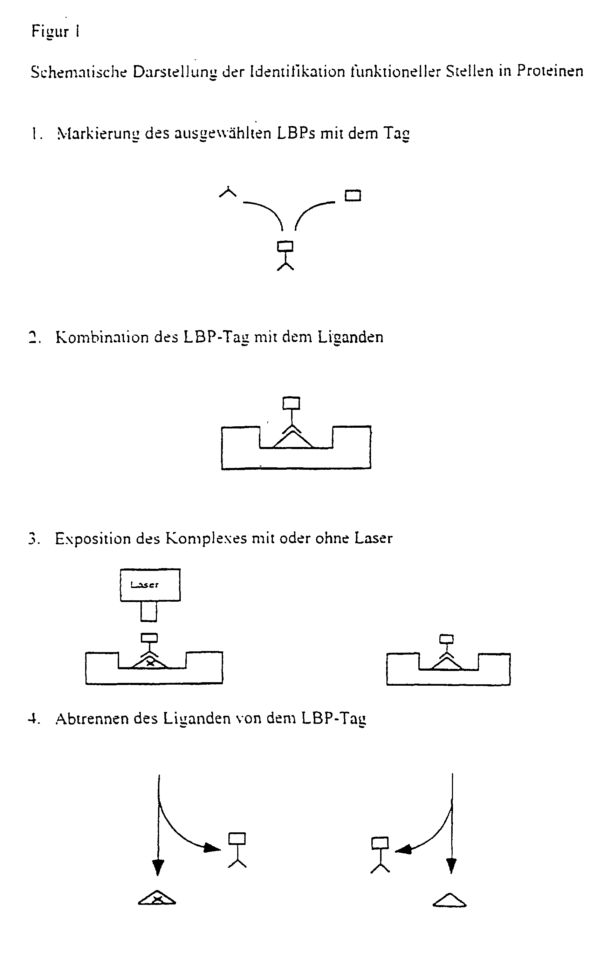

Die Figur 1 zeigt das Schema zur Identifikation funktioneller Stellen in Proteinen.

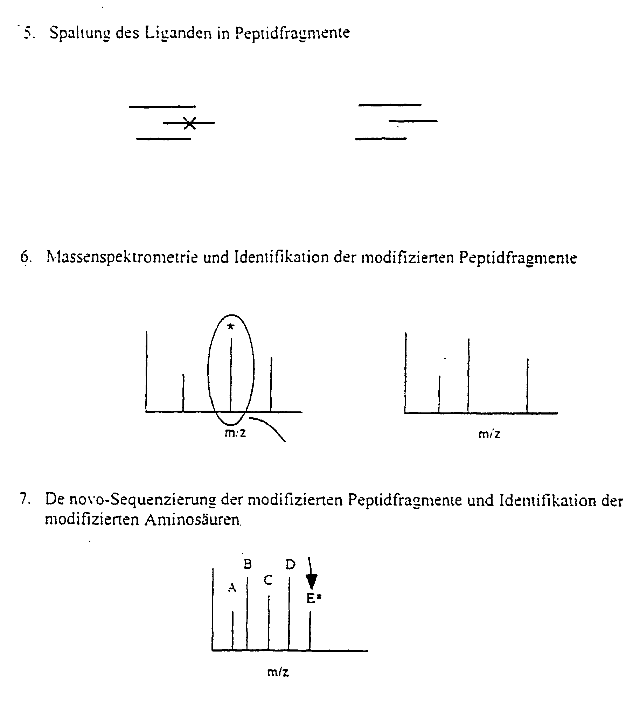

Die Erfindung betrifft auch eine Vorrichtung zur Durchführung des erfindungsgemäßen Verfahrens. Diese ist in Figur 2 gezeigt. Diese Vorrichtung ist ein automatisiertes System integrierter unabhängiger Einheiten/Teile, die zur Identifikation funktioneller Stellen in Proteinen verwendet werden können. Teil A betrifft die Herstellung des LBP-Tags. Eine automatisierte LBP-Screeningmaschine ergibt spezifische LBPs, die gegen spezielle Zielmoleküle/Liganden gerichtet sind, während die Chromophor-Synthesevorrichtung Chromophore der Wahl produziert. Die ausgewählten LBPs und die synthetisierten Chromophore werden chemisch in einer LBP-Chromophor-Kopplungsvorrichtung verknüpft, wodurch der LBP-Tag erhalten wird. Dieser LBP-Tag wird in eine Beladungsvorrichtung überführt, die den LBP-tag in vorbestimmte Kavitäten überführt, die mit dem Zielmolekül/Liganden in der Assay-Platform beschichtet sind.
Ein Transferroboter bewegt dann die Assay-Platform in das Laser-System, um den zweiten Teil B zu initiieren. Die Proben werden mit dem Laser in der gewünschten Wellenlänge bestrahlt, um eine Modifikation durch freie Radikale zu induzieren. Die bestrahlten Proben werden dann durch einen Probentransferroboter in eine LBP-Tag/Ligand-Trennvorrichtung überführt, um den Liganden zu isolieren. Der Ligand wird dann in einer Proteinspalt-Vorrichtung gespalten. Die Peptidfragmente werden dann mit einem Massenspektrometer analysiert, um Veränderungen in der Masse nachzuweisen, oder um eine direkte Sequenzierung durchzuführen. Daten aus den Massenspektren werden dann für die Analyse in der Datenbank verwendet, was schließlich zur Identifikation der Aminosäuren oder Peptidfragmente führt. Alle Teile der Vorrichtung sind mit einem Zentralcomputersystem zur Kontrolle und Analyse verbunden.

## Patentansprüche

1. Verfahren zur Identifikation einer oder mehrerer funktioneller Stellen in einem Protein, **dadurch gekennzeichnet, daß** man
a) das Zielprotein mit einem mit einem laseraktivierbaren Marker (Tag) verknüpften Bindungspartner (BP-Tag) in Kontakt bringt, wobei sich ein Komplex aus Zielprotein und BP-Tag bildet,
b) den Komplex aus Zielprotein und BP-Tag mit Laserlicht bestrahlt, wodurch freie Radikale erzeugt werden, die das gebundene Zielprotein an der Bindungsstelle selektiv verändern, und
c) die selektiv veränderte Region des Proteins durch eine Kombination aus Proteinspaltung und Massenspektrometrie identifiziert.

2. Verfahren nach Anspruch 1, **dadurch gekennzeichnet, daß** man weiterhin die selektiv veränderte Region des Proteins durch de novo-Sequenzierung identifiziert.

3. Verfahren nach einem der Ansprüche 1 oder 2, **dadurch gekennzeichnet, daß** der Bindungspartner für das Protein ausgewählt wird aus dsFv, scFv, Fab, Diabody, immunglobulinartigen Molekülen, Peptiden, RNA, DNA, PNA und kleinen organischen Molekülen.

4. Verfahren nach Anspruch 3, **dadurch gekennzeichnet, daß** der Bindungspartner aus einer kombinatorischen Bank abgeleitet ist.

5. Verfahren nach einem der Ansprüche 1 bis 4, **dadurch gekennzeichnet, daß** der laseraktivierbare Marker eine Substanz ist, die an ein Protein gebunden werden kann und bei Bestrahlung mit Laserlicht freie Radikale erzeugt.

6. Verfahren nach Anspruch 5, **dadurch gekennzeichnet, daß** der Marker AMCA-S, AMCA, BODIPY und Varianten davon, Cascade Blue, Cl-NERF, Dansyl, Dialkylaminocumarin, 4',5'Dichlor-2',7'-dimethoxyfluorescein, DM-NERF, Eosin, Eosin F3S, Erythrosin, Hydroxycumarin, Isosulfan blue, Lissamine Rhodamin B, Malachitgrün, Methoxycumarin, Naphthofluorescein, NBD, Oregon Green 488, 500, 514, PyM-PO, Pyrol, Rhodamin 6G, Rhodamin Green, Rhodamin Red, Rhodol green, 2',4',5',7'-Tetrabromsulfonfluorescein, Tetramethylrhodamin, Texas Red oder X-Rhodamin ist.

7. Verfahren nach einem der Ansprüche 1 bis 6, **dadurch gekennzeichnet, daß** die Proteinspaltung chemisch oder enzymatisch durchgeführt wird.

8. Verfahren nach einem der Ansprüche 1 bis 7, **dadurch gekennzeichnet, daß** die Massenspektrometrie Tandem - Massenspektrometrie, MALDI-TOF ist.

9. Vorrichtung zur Durchführung des Verfahrens nach einem der Ansprüche 1 bis 8, **dadurch gekennzeichnet, daß** sie ein automatisiertes System integrierter unabhängiger Einheiten/Teile ist, die zur Identifikation funktioneller Stellen in Proteinen verwendet werden können, und die folgenden Bestandteile umfaßt:
- eine automatisierte LBP-Screeningmaschine zur Erzeugung spezifischer LBPs, die gegen spezielle Zielmoleküle/Liganden gerichtet sind,
- eine Chromophor-Synthesevorrichtung zur Produktion von Chromophoren,
- eine LBP-Chromophor-Kopplungsvorrichtung zur Verknüpfung der ausgewählten LBPs und der synthetisierten Chromophore,
- eine Beladungsvorrichtung zur Überführung des LBP-Tag in vorbestimmte Kavitäten, die mit dem Zielmolekül/Liganden in der Assay-Platform beschichtet sind,
- einen Transferroboterarm zur Bewegung der Assay-Platform in das Laser-System,
- einen Probentransferroboter zur Bewegung der Proben in eine LBP-Tag/Ligand-Trennvorrichtung,
- eine Proteinspalt-Vorrichtung,
- ein Massenspektrometer,
- eine Datenbank,
- ein Zentralcomputersystem.

## Claims

1. A method for identifying one or more functional sites in a protein, **characterized in that**
a) the target protein is contacted with a binding partner linked to a laser-activatable marker (tag), (BP-tag), to form a complex of target protein and BP-tag,
b) the complex of target protein and BP-tag is irradiated with laser light, whereby free radicals are generated which selectively alter the bound target protein at the binding site, and
c) the selectively altered region of the protein is identified by a combination of protein cleavage and mass spectrometry.

2. The method according to claim 1 or 2, **characterized in that** in addition the selectively altered region of the protein is identified by de novo sequencing.

3. The method according to claim 1, **characterized in that** the binding partner for the protein is selected from the group consisting of dsFv, scFv, Fab, diabody, immunoglobulin-like molecules, peptides, RNA, DNA, PNA and small organic molecules.

4. The method according to claim 3, **characterized in that** the binding partner is derived from a combinatorial library.

5. The method according to any of claims 1-4, **characterized in that** the laser-activatable marker is a substance which can be bound to a protein and generates free radicals on irradiation with laser light.

6. The method according to claim 5, **characterized in that** the marker is selected from the group consisting of AMCA-S, AMCA, BODIPY and variants thereof, Cascade Blue, Cl-NERF, dansyl, dialkylaminocoumarin, 4',5'-dichloro-2',7'-dimethoxyfluorescein, DM-NERF, eosin, eosin F3S, erythrosin, hydroxycoumarin, Isosulfan Blue, lissamine rhodamine B, malachite green, methoxycoumarin, naphthofluorescein, NBD, Oregon Green 488, 500, 514, PyMPO, pyrene, Rhodamine 6G, Rhodamine Green, Rhodamine Red, Rhodol Green, 2',4',5',7'-tetrabromosulphonefluorescein, tetramethylrhodamine, Texas Red and X-rhodamine.

7. The method according to any of claims 1-6, **characterized in that** the protein cleavage is carried out chemically or enzymatically.

8. The method according to any of claims 1-7, **characterized in that** the mass spectrometry is tandem mass spectrometry, MALDI-TOF.

9. Apparatus for carrying out the method according to any of claims 1 to 8, **characterized in that** it is an automated system of integrated independent units/parts which can be used for identifying functional sites in proteins, and which comprises the following constituents:
- an automated LBP screening machine for generating specific LBPs which are directed against specific target molecules/ligands,
- a chromophore synthesizing apparatus for producing chromophores,
- an LBP-chromophore coupling apparatus for linking the selected LBPs and the synthesized chromophores,
- a loading apparatus for transferring the LBP-tag into predetermined wells which are coated with the target molecule/ligand in the assay platform,
- a transfer robot arm for moving the assay platform into the laser system,
- a sample transfer robot for moving the samples into an LBP-tag/ligand separating apparatus,
- a protein cleaving apparatus,
- a mass spectrometer,
- a database,
- a central computer system.

## Revendications

1. Procédé pour l'identification d'un ou plusieurs sites fonctionnels dans une protéine, **caractérisé en ce que**
a) on met en contact la protéine cible avec un partenaire de liaison (BP-Tag) lié à un marqueur (Tag) activable par laser, avec formation d'un complexe de la protéine cible et du BP-Tag,
b) on expose le complexe de la protéine cible et du BP-Tag à une lumière laser, ce qui produit des radicaux libres, qui modifient d'une manière sélective la protéine cible liée au site de liaison, et
c) on identifie par une combinaison d'une dissociation de la protéine et d'une spectrométrie de masse la région de la protéine ayant subi une modification sélective.

2. Procédé selon la revendication 1, **caractérisé en ce que** la région de la protéine ayant subi une modification sélective est en outre identifiée par un séquençage de novo.

3. Procédé selon l'une des revendications 1 ou 2, **caractérisé en ce que** le partenaire de liaison de la protéine est choisi parmi le dsFv, le scFv, le Fab, le Diabody, les molécules de type immunoglobuline, les peptides, l'ARN, l'ADN, l'APN et les petites molécules organiques.

4. Procédé selon la revendication 3, **caractérisé en ce que** le partenaire de liaison est obtenu à partir d'une banque combinatoire.

5. Procédé selon l'une des revendications 1 à 4, **caractérisé en ce que** le marqueur activable par laser est une substance qui peut se lier à une protéine et qui lors de son exposition à la lumière laser produit des radicaux libres.

6. Procédé selon la revendication 5, **caractérisé en ce que** le marqueur est l'AMCA-S, l'AMCA, le BODIPY et ses variantes, le Cascade Blue, le Cl-NERF, le Dansyl, une dialkylaminocoumarine, la 4',5'-dichloro-2',7'-diméthoxyfluorescéine, le DM-NERF, l'éosine, l'éosine F3S, l'érythrosine, l'hydroxycoumarine, le bleu isosulfan, la lissamine-rhodamine B, le vert malachite, la méthoxycoumarine, la naphtofluorescéine, le NBD, l'orégon Green 488, 500, 514, le PyM-PO, le pyrrole, la rhodamine 6G, le vert de rhodamine, le rouge de rhodamine, le vert rhodol, la 2,4',5',7'-tétrabromosulfofluorescéine, la tétraméthylrhodamine, le rouge Texas ou la rhodamine X.

7. Procédé selon l'une des revendications 1 à 6, **caractérisé en ce que** la dissociation de la protéine est mise en oeuvre par voie chimique ou enzymatique.

8. Procédé selon l'une des revendications 1 à 7, **caractérisé en ce que** la spectrométrie de masse est une spectrométrie de masse en tandem, MALDI-TOF.

9. Appareillage pour mettre en oeuvre le procédé selon l'une des revendications 1 à 8, **caractérisé en ce qu'**il s'agit d'un système automatisé d'unités/parties éléments intégrés indépendants, qui peuvent être utilisé pour l'identification de sites fonctionnels dans des protéines, et qui comprend les constituants suivants :
- une machine automatique de criblage des LBP, pour produire des LBP spécifiques qui sont dirigés contre des molécules cibles/ligands spéciaux,
- un appareil de synthèse de chromophore pour produire des chromophores,
- un appareil de couplage LBP-chromophore, pour lier les LBP sélectionnés et les chromophore synthétisés,
- un dispositif de chargement, pour placer le marqueur LBP dans des cavités prédéfinies, qui sont revêtues de la molécule cible/ligand dans la plateforme d'analyse,
- un bras robot de transfert, pour déplacer la plateforme d'analyse dans le système laser,
- un robot de transfert d'échantillon, pour déplacer les échantillons dans un dispositif de séparation marqueur LBP/ligand,
- un appareil de dissociation des protéines,
- un spectromètre de masse,
- une banque de données,
- un système informatisé central.
